# EUROPEAN PATENT SPECIFICATION

(11) **EP 2 020 234 B1**
(45) Date of publication and mention of the grant of the patent: **25.04.2012**
(21) Application number: 06783810.2
(22) Date of filing: 27.07.2006
(51) Int. Cl.: A61K 31/5375, A61K 31/695, A61P 1/14, A61P 1/04

(54) **SYNERGIC COMBINATION OF PINAVERIUM BROMIDE AND DIMETHICONE OR SIMETHICONE**
SYNERGISTISCHE KOMBINATION VON PINAVERIUM BROMID UND DIMETHICON ODER SIMETHICON
COMBINAISON SYNERGIQUE DE BROMURE DE PINAVERIUM ET DE DIMETHICONE OU SIMETHICONE

(30) Priority: 09.03.2006 MX PA06002746
(43) Date of publication of application: 04.02.2009
(73) Proprietor: World Trade Import-Export, WTIE A.G., 6302 Zug (CH)
(72) Inventor: GARCÍA ARMENTA, María Elena, C.P. 45020 Guadalajara, Jalisco, México (MX); ALVAREZ OCHOA, Victor Guillermo, C.P. 45222 Zapopan, Jalisco, México (MX); SANTOS MURILLO, Josefina, C.P. 44600 Zapopan, Jalisco (MX); GARCÍA ARMENTA, Patricia, C.P. 45110 Zapopan, Jalisco (MX); ESPINOSA ABDALA, Leopoldo de Jesús, C.P. 45110 Zapopan, Jalisco, México (MX)
(74) Representative: Carvajal y Urquijo, Isabel
(86) International application number: PCT/MX2006/000081
(87) International publication number: WO 2007/102725

(56) References cited:
- US-A1- 2002 193 413
- DATABASE BIOSIS [Online] March 2003 SHEN MIN-NING ET AL.: 'Pinaverium Bromide combined with bifid-triple viable power for treatment of irritable bowel syndrome', XP008116613 Database accession no. (200300241834)
- DATABASE BIOSIS [Online] AWAD R. ET AL.: 'Irritable bowel syndrome treatment using pinaverium bromide as a calcium channel blocker', XP008116614 Database accession no. (199698560254) & ACTA GASTROENTEROLOGICA LATINOAMERICANA vol. 25, 1995, pages 137 - 144

## Description

### FIELD OF THE INVENTION

The present invention generally relates to pharmaceutical industry and to drug preparer pharmaceutical industry for prevention and/or treatment of irritable bowel syndrome (IBS) and characteristic symptoms such as abdominal discomfort or pain associated with changes in defecation and frequently abdominal distension, in particular.

### BACKGROUND OF THE INVENTION

The history of this pathology goes back to the XIX century, with publications such as those from Powell (1820), Howship (1830), Cumming (1849), Osler (1892). Hurst, en 1921, mentioned the mucous colitis; Bockus, in 1928, the neurogenic mucous colitis; Tyle, in 1928, the colonic spasm. In 1929, Jordan and Kiefer mentioned for the first time the "Irritable Colon"

In 1966, Chaudhary and Truelove coined the term "Irritable Colon Syndrome", DeLor, the "Irritable Bowel Syndrome (IBS)". Tompson et al, in 1999, referred to SII as a group of functional disorders wherein abdominal pain is associated with changes in the underlying bowel habit and with disordered or altered defecation characteristics.

During the period comprised between the end of the XIX century and the beginning of the XX century, the physicians' interest on a series of non-organic base disorders starts. At that time, references about abdominal symptoms slightly characterized started to be found and which receive names such as: "spasmodic colon, unstable colon, nervous colon, neurogenic mucous colitis, nervous colitis, mucous colitis, spasmodic colitis, functional colopathy" and the like, generally tending to define this disease as non-organic and highly related to the psychic sphere.

During the 80's, the inscription of the so-called "Irritable Colon" within the widest concept of "Irritable Bowel Syndrome (IBS)" is reached, and the decisive steps toward its clinical characterization which represent the successive enumeration of diagnostic criterion are made. Traditionally, until then the Irritable Bowel Syndrome diagnosis had been made through discard, being believed that in order to identify it any organic disease which could have the same symptoms should be excluded.

Currently, it is known that the Irritable Bowel Syndrome (IBS) is the most significant cause of intestinal diseases in the community, being the most common reason for medical visits by being presented in the patient the most common symptoms. The Irritable Bowel Syndrome (IBS) is a common clinical problem, maintaining a high prevalence in the general population from 14 to 20% in women and from 5 to 19% in men.

The Irritable Bowel Syndrome (IBS) is a chronic and recurrent functional disorder, manifested through a set of gastrointestinal signs and symptoms caused by a bowel dysfunction, wherein there is not a structural lesion of the bowel which causes the symptoms. The most prominent symptoms are: abdominal pain or distension, tumescence and alteration of the underlying bowel habits, constipation, diarrhea or alternation thereof, as per each case. The active episodes of the symptoms are followed by periods of relative inactivity.

Base on the predominant symptoms, the Irritable Bowel Syndrome (IBS) is divided in three subtypes: constipation-predominant Irritable Bowel Syndrome, (**C-IBS**), diarrhea-predominant Irritable Bowel Syndrome (**D-IBS**), and constipation alternating with diarrhea Irritable Bowel Syndrome **(A-IBS).**

There are many causes which propitiate the development of Irritable Bowel Syndrome (IBS), such as: a disorder in the bowel smooth muscular movement or a slow tolerance to constipation and movement of the bowel, without existing any anomaly in the bowel structure; a low-fiber diet; emotional stress; use of laxatives; an attack of infectious diarrhea or another temporal bowel inflammation. This condition is presented at any age, but it often starts in adolescence and the beginning of the adult life, occurring more frequently in women.

So far, the use of medicaments in the art is only intended for the treatment of the predominant symptom in each case, taking into account the intensity and frequency of the symptoms, being the afore-mentioned a motive so that during treatment of IBS various drugs are administered made for providing relief for multiple symptoms which said disease presents.

Shen Min-Ning describes a clinical study aimed to compare the curative effect of pinaverium bromide in combination with a triple viable energy bifid vs. pinaverium bromide alone for the treatment of IBS (SHEN Min-ning, CHEN Zhi-tan, "Pinaverium bromide combined with bifid-triple viable power for treatment of irritable bowel sindrome", 2003, Medical Department of Digestive Diseases, the First Hospital of Nanjing Affiliated with the Nanjing Medical University, Nanjing 210006, China). Results revealed that the healing effect on patients in the treated group was 98%, while on the control group reached only the 80% (P> 0.05). The curative effect with respect to diarrhea in treated patients was better than in the control group. It was concluded that the combination of bromide Pinaverium with bifid triple viable energy has proven effective in treating IBS.

Further, Award disclosed a randomized, double-blind, placebo-controlled study, conducted with 40 patients with irritable bowel syndrome who received 50 mg Pinaverium bromide or placebo, orally three times daily with food (Award R. et al., "Irritable bowel syndrome treatment using pinaverium bromide as a calcium channel blocker. A randomized double-blind placebo-controlled trial ", 1995, Experimental Medicine Unit U-404-B, Hospital General de Mexico, Mexico). Results showed that pinaverium bromide treated patients reduced their pain from several hours to a few minutes, and the authors concluded that the said treatment is safe and produces significant benefits in quality of life of patients, considering it as an effective drug for the treatment of patients with IBS.

Before the great arsenal of drugs which invaded the market and the physicians' consulting rooms for treatment of Irritable Bowel Syndrome (IBS), and with the object of suppressing the drawbacks mentioned above, the development of the pharmaceutical composition which protection thereof is pretended through the present application was carried out, composed by the combination of an L-type calcium channels blocking substance with selective action on the smooth muscle fiber of the gastrointestinal tract such as Pinaverium Bromide and a linear polymer of methylated siloxanes (inert silicone) such as Dimeticone or Simeticone, formulated in a single dosage unit.

**Pinaverium Bromide** is a derivative of the quaternary ammonium of chemical name 4-(6-bromoveratril)-4-{2-[2-(6,6 dimethyl-2-norpinil)etoxyl]-ethyl}morfolinium bromide, whose chemical formula is: C26H41Br2NO4 and its molecular weight is: 591.4. And whose structural formula is the following:

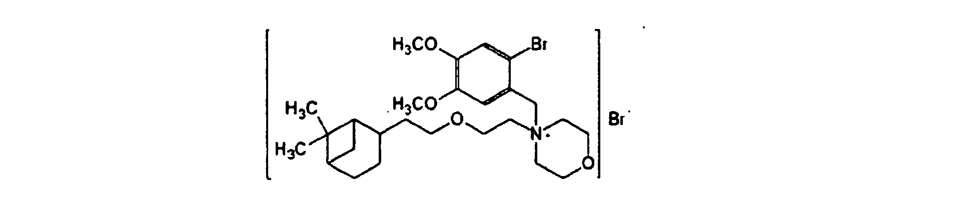

Pinaverium Bromide is a non-atrophic, anticholinergic, antispasmodic agent with a potent musculotropic action and is a very weak neurotropic component. Pinaverium Bromide is a calcium channel blocker with selective action on the smooth muscle fiber of the gastrointestinal tract which achieves the relief of abdominal pain and correction of the altered gastrointestinal passage. It is also able to decrease the quantity of acid without modifying the volume of gastric secretion and accelerating evacuation of stomach. Due to the quaternary ammonium group contained in its chemical structure, Pinaverium Bromide has a high polarity, so its passage through lipid membranes is limited.

**Dimeticone** or **Simeticone** corresponds to the chemical name of:: trimethyl-trimethylsililoxy-silane and its chemicals formula: C₆H₁₈OSi₂ whose structural formula corresponds to the monomer:

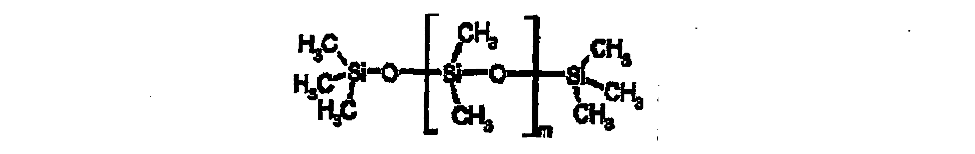

Chemically is a lineal polymer of methylated siloxanes (inert silicone), with a number of units between 200 and 350 depending on its viscosity. Dimeticone or Simeticone is an antiflatulence and defrothing agent active orally which is used to relieve pain and abdominal troubles caused by the pressure of an excess of gasses. Due to the fact that dimeticone or simeticone is an inert molecule, it cannot be absorbed since it is not transformed by intestinal flora, so it is expelled through feces. It has surface-active properties which decrease the surface tension of mucogaseous bubbles, responsible for gas retention, thus allowing its disintegration and avoiding its formation, originating a carminative and antiflatulence effect. Interactions of dimeticone with other drugs or herbs have not been communicated. The adverse effects of dimeticone are, in general, mild and transitory. The most frequent adverse effect is moderated constipation.

### OBJECTS OF THE INVENTION

One of the objects of the present invention is to achieve a pharmaceutical composition proper for treatment or prevention of Irritable Bowel Syndrome (IBS) with fewer amounts of dosage of the formulation components than those from the art state.

Another of the objects is to achieve the previous objective by conferring the medicament a greater speed of action in connection with the formulations already known.

Still another object of the present invention is to have a formula which has minor secondary effects than those currently presented by using existent medicaments.

Other objects and advantages of the present invention would be apparent from the study of the following description and the drawings accompanying with illustrative purposes only rather than limitative.

### BRIEF DESCRIPTION OF THE INVENTION

Shortly, the present invention is based in the combination of molecules from the public domain which have already proved its efficiency for treatment of symptomatology related to Irritable Colon Syndrome, but which combined provide unnoticeable with a significant synergic effect.

The present invention describes a pharmaceutical composition composed by the combination of Pinaverium Bromide, an L-type calcium channels blocking substance with selective action on the smooth muscle fiber of the gastrointestinal tract, and Dimeticone or Simeticone, a linear polymer of methylated siloxanes (inert silicone), which is useful for prevention and/or treatment of Irritable Bowel Syndrome (IBS) and characteristic symptoms such as abdominal discomfort or pain associated with changes in defecation and frequently abdominal distension.

The combination of the active principles mentioned above causes a synergic effect when they are jointly administered than when they are independently administered, providing benefits such as: fewer dosages of each of the actives, greater speed of action and fewer side effects.

### DETAILED DESCRIPTION OF THE INVENTION

The present invention is applied to the pharmaceutical industry field and has the aim of offering a treatment for Irritable Bowel Syndrome (IBS) through the administration of a pharmaceutical composition which fulfill the object of relieving the manifested symptoms, in addition to blocking the calcium channels in order to modify and/or correct the bowel contractile activity with selective action on the smooth muscular fiber of the gastrointestinal tract and decrease the quantity of acid without modifying the volume of gastric secretion and accelerating evacuation of the stomach.

Thus, the present invention is the synergic combination composed by Pinaverium Bromide as a type-L calcium channels blocking agent with selective action on a smooth muscular fiber of the gastrointestinal tract, and Dimeticone or Simeticone as an antiflatulence agent, as well as pharmaceutically acceptable excipients.

The development of the pharmaceutical composition, object of the present invention, was carried out by combining different active principles with specific therapeutic activity, among which there is a calcium channel, non-atrophic, antispasmodic blocking agent known as Pinaverium Bromide and an antiflatulence agent known as Dimeticone or Simeticone, which produce a synergic effect when they are jointly administered in a single dosage unit than when they are independently administered, providing benefits such as: a decrease in the number of dosages of each of the actives, greater speed of action and fewer side effects in patients suffering from Irritable Bowel Syndrome (IBS).

In order to evaluate the efficiency and tolerance of the pharmaceutical composition reason of the present invention, as well as the synergic effect of the combined active principles, different comparative assays where performed by administering different quantities of the active principles Pinaverium Bromide and Dimeticone or Simeticone, which are hereinafter more broadly described.

### EXAMPLES

### EXAMPLE 1:

A comparative, clinical study was performed in 100 patients divided in two groups, who suffer from Irritable Colon Syndrome, manifesting predominant diarrhea.

After basal evaluations of the total colonic passage time, the patients with Irritable Colon Syndrome were randomized for administer them the treatment as described below: Group 1: Pinaverium Bromide + Dimeticone twice a day during 2 weeks. Group 2: only Pinaverium Bromide twice a day during 2 weeks. The registered parameters were: Defecation and colonic passage time, being evaluated the serum levels of substance (P) and neuropeptides (Y) before and after the treatment.

### Results:

The frequency in daily defecation was significantly reduced after the treatment for group 1 (Pinaverium Bromide + Dimeticone, 3.5 ± 2.2 vs. 2.9 ± 1.2) than with administration of Pinaverium Bromide alone (P<0.05). The consistency of the feces became well formed after the treatment administered to Group 1.

The combination of active principles improved the global symptoms in patients suffering from Irritable Colon Syndrome in an 87.8% compared with a 73.4% obtained with the administration of Pinaverium Bromide alone. The total colonic passage time was significantly extended after treatment with Pinaverium Bromide + Dimeticone (24.0 ± 16.5 hours vs. 21.4' ± 15.5) than with the administration of Pinaverium Bromide alone (P<0.001). Neither the levels of substance P nor the level of neuropeptide Y were significantly varied after the treatment.

### Conclusions:

With the combination of Pinaverium Bromide + Dimeticone better effects on the signs and symptoms manifested in patients suffering from Irritable Colon Syndrome were obtained compared with those obtained with the administration of Pinaverium Bromide alone, without reporting adverse events.

### EXAMPLE 2:

A comparative, clinical study was performed in 100 patients divided in two groups, who suffer from Irritable Colon Syndrome, manifesting predominant diarrhea.

After basal assessment of the total colonic passage time, the patients with Irritable Colon Syndrome were randomized for administering them the treatment as described below: Group 1: Pinaverium Bromide + Dimeticone twice a day during 2 weeks. Group 2: only Dimeticone thrice a day during 2 weeks. The registered parameters were: Defecation and Colonic passage time, being evaluated the serum levels of substance (P) and neuropeptides (Y) before and after the treatment.

### Results:

The frequency in daily defecation was significantly reduced after the treatment for Group 1 (Pinaverium Bromide + Dimeticone, 3.7 ± 2.2 vs. 2.8 ± 1.3) than with administration of Dimeticone or Simeticone alone (P<0.03). The consistency of the feces became well formed after the treatment administered to Group 1.

The combination of active principles improved the global symptoms in patients suffering from Irritable Colon Syndrome in an 88.9% compared with a 73.2% obtained with the administration of Dimeticone or Simeticone alone (P < 0.04). The total colonic passage time was significantly extended after treatment with Pinaverium Bromide + Dimeticone (24.5 ± 17.4 hours vs. 20.3 ± 15.3) than with the administration of Dimeticone or Simeticone alone (P<0.001). Neither the levels of substance P nor the level of neuropeptide Y were significantly varied after the treatment.

### Conclusions:

With the combination of Pinaverium Bromide + Dimeticone better effects on the signs and symptoms manifested in patients suffering from Irritable Colon Syndrome were obtained compared with those obtained with the administration of Dimeticone or Simeticone alone, being reported 15 adverse events in Group 2, which were manifested with abdominal distension, flatulences and meteorism not meriting however the suspension of the treatment.

## Claims

1. Synergic combination **characterized in that** said combination is composed by Pinaverium Bromide as a type-L calcium channels blocking agent with selective action on a smooth muscular fiber of the gastrointestinal tract and Dimeticone or Simeticone as an antiflatulence agent, as well as pharmaceutically acceptable excipients.

2. Synergic combination according to claim 1, **characterized in that** said combination is formulated in one single dosage unit.

3. Synergic combination according to any of claims 1 or 2, **characterized in that** the active principle Pinaverium Bromide is present in the formulation in a concentration range from 30 to 120 mg per dosage unit.

4. Synergic combination according to claim 3, **characterized in that** the active principle Pinaverium Bromide is present in a concentration of 100 mg per dosage unit.

5. Synergic combination according to any of claims 1 or 2, **characterized in that** the active principle Dimeticone or Simeticone is present in the formulation in a concentration range from 20 to 120 mg per dosage unit.

6. Synergic combination according to claim 5, **characterized in that** the active principle Dimeticone or Simeticone is present in the formulation in a concentration of 100 mg per dosage unit.

7. Synergic combination according to anyone of the preceding claims for the prevention and/or treatment of Irritable Bowel Syndrome and characteristic symptoms, wherein said symptoms are abdominal discomfort or pain associated with changes in defecation or abdominal distension.

## Patentansprüche

1. Synergetische Kombination **dadurch gekennzeichnet, dass** die genannte Kombination aus Folgendes besteht: Pinaverium Bromid als L-Typ-Calcium-Kanal-Blocker mit selektiver Wirkung auf die glatte Muskelfaser des Magen-Darm-Traktes und Dimeticon oder Simeticon als Blähungsmittel, sowie pharmazeutisch annehmbare Hilfsstoffe.

2. Synergetische Kombination nach Anspruch 1, **dadurch gekennzeichnet, dass** die genannte Kombination in einer einzigen Dosierungseinheit formuliert ist.

3. Synergetische Kombination nach einem der Ansprüche 1 oder 2, **dadurch gekennzeichnet, dass** der Wirkstoff Pinaverium Bromid in der Formulierung in einem Konzentrationsbereich von 30 bis 120 mg pro Dosierungseinheit enthalten ist.

4. Synergetische Kombination nach Anspruch 3, **dadurch gekennzeichnet, dass** der Wirkstoff Pinaverium Bromid in einer Konzentration von 100 mg pro Dosierungseinheit enthalten ist.

5. Synergetische Kombination nach einem der Ansprüche 1 oder 2, **dadurch gekennzeichnet, dass** der Wirkstoff Dimeticon oder Simeticon in der Formulierung in einem Konzentrationsbereich von 20 bis 120 mg pro Dosierungseinheit enthalten ist.

6. Synergetische Kombination nach Anspruch 5, **dadurch gekennzeichnet, dass** der Wirkstoff Dimeticon oder Simeticon in der Formulierung in einem Konzentrationsbereich von 100 mg pro Dosierungseinheit enthalten ist.

7. Synergetische Kombination nach einem der vorangehenden Ansprüche für die Vorbeugung und/oder Behandlung vom Reizdarmsyndrom und kennzeichnende Symptome, wobei die genannte Symptome Unterleibbeschwerden oder mit Bauchdehnung oder einer Änderung des Stuhlganges verbundenen Schmerzen.

## Revendications

1. Combinaison synergique **caractérisée en ce que** ladite combinaison est composée de bromure de pinavérium comme un agent bloquant des canaux calciques de type L avec une action sélective sur une fibre musculaire lisse du tractus gastro-intestinal et diméticone ou siméticone comme agent antiflatulence, ainsi que des excipients pharmaceutiquement acceptables.

2. Combinaison synergique selon la revendication 1, **caractérisée en ce que** ladite combinaison est formulée en une seule unité de dosage.

3. Combinaison synergique selon l'une quelconque des revendications 1 ou 2, **caractérisée en ce que** le principe actif, le bromure de pinavérium, est présent dans la formulation à une concentration allant de 30 a 120 mg par unité de dosage.

4. Combinaison synergique selon la revendication 3, **caractérisée en ce que** le principe actif, le bromure de pinavérium, est présent à une concentration de 100 mg par unité de dosage.

5. Combinaison synergique selon l'une quelconque des revendications 1 ou 2, **caractérisée en ce que** le principe actif, diméticone ou siméticone, est présent dans la formulation à une concentration allant de 20 a 120 mg par unité de dosage.

6. Combinaison synergique selon la revendication 5, **caractérisée en ce que** le principe actif, diméticone ou siméticone, est présent dans la formulation à une concentration de 100 mg par unité de dosage.

7. Combinaison synergique selon l'une quelconque des revendications antérieures pour la prévention et/ou le traitement du syndrome du colon irritable et des symptômes caractéristiques, dans laquelle lesdits symptômes sont malaise abdominal ou douleur associée aux changements lors de la défécation ou de la distension abdominale.
